# FASCICULE DE BREVET EUROPEEN

(11) **EP 0 682 708 B1**
(45) Date de publication et mention de la délivrance du brevet: **22.09.2004**
(21) Numéro de dépôt: 95903829.0
(22) Date de dépôt: 13.12.1994
(51) Int. Cl.: C12N 15/49, C07K 14/16, A61K 48/00, A61K 39/21, C12N 15/86, C12N 5/10

(54) **COMPOSITION DE VARIANTS TRANSDOMINANTS DE PROTEINES VIRALES POUR UN EFFET ANTIVIRAL**
ZUSAMMENSETZUNG VON TRANSDOMINANTEN VARIANTEN VON VIRUS- PROTEINEN ZUR ANTIVIRALEN WIRKUNG
ANTIVIRAL COMPOSITION OF VIRAL PROTEIN TRANS-DOMINANT VARIANTS

(30) Priorité: 13.12.1993 FR 9314914
(43) Date de publication de la demande: 22.11.1995
(73) Titulaire: TRANSGENE S.A., 67000 Strasbourg (FR)
(72) Inventeur: MEHTALI, Majid, F-67400 Illkirch-Graffenstaden (FR); GUSS, Tania, F-67330 Dossenheim-sur-Zinsel (FR)
(74) Mandataire: Ahner, Francis
(86) Numéro de dépôt international: PCT/FR1994/001457
(87) Numéro de publication internationale: WO 1995/016780

(56) Documents cités:
- EP-A- 0 406 557
- JOURNAL OF VIROLOGY, vol.67, no.6, Juin 1993 pages 3199 - 3207 BAHNER, I. ET AL. 'Comparison of trans-dominant inhibitory mutant Human Immunodeficiency virus type 1 genes expressed by retroviral vectors in human T lymphocytes'
- HUMAN GENE THERAPY, vol. 4, 1993, pages 625-634, LIEM S.E. ET AL. "The development and testing of retroviral vectors expressing trans-dominant mutants of HIV-1 proteins to confer anti-HIV-1 resistance"
- CELL, vol. 58, pages 215-223, 1992, GREEN M. ET AL. "Mutational analysis of HIV-1 Tat minimal domain peptides: identification of trans-dominant mutants that suppress HIV-LTR-driven gene expression"

### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 22 58 07
   (ii) TITRE DE L' INVENTION: Nouvelle composition pour un effet antiviral
   (iii) NOMBRE DE SEQUENCES: 7
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 86 acides aminés
      (B) TYPE: acide aminé
      (D) CONFIGURATION: linéaire
   (ii) TYPE DE MOLECULE: protéine
   (iii) HYPOTHETIQUE: NON
   (iii) ANTI-SENS: NON
   (vi) ORIGINE:
      (A) ORGANISME: Human immunodeficiency virus type 1
      (B) SOUCHE: Lai
      (C) INDIVIDUEL ISOLE: sequence de la proteine TAT du VIH-1
   (xi) DESCRIPTION DE LA SEQUENCE: SEQ ID NO: 1:
   Met Glu Pro Val Asp Pro Arg Leu Glu Pro Trp Lys His Pro Gly Ser
   1               5                   10                  15
   Gln Pro Lys Thr Ala Cys Thr Thr Cys Tyr Cys Lys Lys Cys Cys Phe
               20                  25                  30
   His Cys Gln Val Cys Phe Thr Thr Lys Ala Leu Gly Ile Ser Tyr Gly
           35                  40                  45
   Arg Lys Lys Arg Arg Gln Arg Arg Arg Pro Pro Gln Gly Ser Gln Thr
       50                  55                  60
   His Gln Val Ser Leu Ser Lys Gln Pro Thr Ser Gln Pro Arg Gly Asp
   65                  70                  75                  80
   Pro Thr Gly Pro Lys Glu
                   85
(2) INFORMATION POUR LA SEQ ID NO: 2:
   (i) CARACTERISTIQUES DE LA SEQUENCE:
      (A) LONGUEUR: 116 acides aminés
      (B) TYPE: acide aminé
      (D) CONFIGURATION: linéaire
   (ii) TYPE DE MOLECULE: protéine
   (iii) HYPOTHETIQUE: NON
   (lii) ANTI-SENS: NON
   (vi) ORIGINE:
      (A) ORGANISME: human immunodeficiency virus type 1
      (B) SOUCHE: Lai
      (C) INDIVIDUEL ISOLE: sequence de la proteine rev du virus VIH-1
   (xi) DESCRIPTION DE LA SEQUENCE: SEQ ID NO: 2:
   Met Ala Gly Arg Ser Gly Asp Ser Asp Glu Asp Leu Leu Lys Ala Val
   1               5                   10                  15
   Arg Leu Ile Lys Phe Leu Tyr Gln Ser Asn Pro Pro Pro Asn Pro Glu
               20                  25                  30
   Gly Thr Arg Gln Ala Arg Arg Asn Arg Arg Arg Arg Trp Arg Glu Arg
           35                  40                  45
   Gln Arg Gln Ile His Ser Ile Ser Glu Arg Ile Leu Ser Thr Tyr Leu
       50                  55                  60
   Gly Arg Ser Ala Glu Pro Val Pro Leu Gln Leu Pro Pro Leu Glu Arg
   65                  70                  75                  80
   Leu Thr Leu Asp Cys Asn Glu Asp Cys Gly Thr Ser Gly Thr Gln Gly
                   85                  90                  95
   Val Gly Ser Pro Gln Ile Leu Val Glu Ser Pro Thr Val Leu Glu Ser
               100                 105                 110
   Gly Thr Lys Glu
           115
(2) INFORMATION POUR LA SEQ ID NO: 3:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (C) INDIVIDUEL ISOLE: oligonucleotide de mutagenese (TAT Phe38 en Asp)
   (xi) DESCRIPTION DE LA SEQUENCE: SEQ ID NO: 3:
   TAAAGCTTTT GTTGTGTCAC AAACTT                                        26
(2) INFORMATION POUR LA SEQ ID NO: 4:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (C) INDIVIDUEL ISOLE: oligonucleotide de mutagenese (REV Gln, Leu en Gly, Ser)
   (xi) DESCRIPTION DE LA SEQUENCE: SEQ ID NO: 4:
   TCTCTCAAGC GGTGGGGATC CAAGAGGCAC AGG 33
(2) INFORMATION POUR LA SEQ ID NO: 5:
   (i) CARACTERISTIQUES DE LA SEQUENCE:
      (A) LONGUEUR: 41 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 5:
   ATCAAGAGTA TCTCTGAATT CCGGTGGGGA TCCAAGAGGC A 41
(2) INFORMATION POUR LA SEQ ID NO: 6:
   (i) CARACTERISTIQUES DE LA SEQUENCE:
      (A) LONGUEUR: 34 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (C) INDIVIDUEL ISOLE: oligonucleotide de synthese (OTG4413)
   (xi) DESCRIPTION DE LA SEQUENCE: SEQ ID NO: 6:
   TCTCTCCACC TTCGCGGCCG CTTCCTTCGG GCCT 34
(2) INFORMATION POUR LA SEQ ID NO: 7:
   (i) CARACTERISTIQUES DE LA SEQUENCE:
      (A) LONGUEUR: 22 paires de bases
      (B) TYPE: acide nucléique
      (C) NOMBRE DE BRINS: simple
      (D) CONFIGURATION: linéaire
   (ii) TYPE DE MOLECULE: ADNc
   (lii) HYPOTHETIQUE: NON
   (iii) ANTI-SENS: NON
   (vi) ORIGINE:
      (C) INDIVIDUEL ISOLE: oligonucleotide de synthese (OTG4370)
   (xi) DESCRIPTION DE LA SEQUENCE: SEQ ID NO: 7:
   GGCATTGAGA GATCTAACAG CA 22

## Description

La présente invention concerne une composition résultant de l'association de variants trans-dominants de deux protéines virales issues du même virus. L'association de ces deux variants permet de conférer une résistance à l'infection ou la propagation du virus en question. La présente invention trouve une application intéressante dans le traitement ou la prévention des infections à VIH (Virus de l'Immunodéficience Humaine) responsable du SIDA (Syndrome d'Immunodéficience Acquise).

Dans le cadre de la recherche et l'élaboration de médicaments inhibiteurs des infections virales, la thérapie génique somatique par immunisation intracellulaire constitue une des approches prometteuses pour l'avenir. Ce concept, défini par Baltimore (1988, Nature, *335*, 395-396), consiste à modifier génétiquement des cellules afin de leur faire synthétiser un acide nucléique ou une protéine hétérologue leur conférant une résistance contre l'infection virale. Une des voies possibles pour acquérir cette résistance est de faire synthétiser à la cellule hôte des gènes inhibiteurs d'une étape du cycle viral. Bien que toutes les étapes du cycle viral puissent être ciblées pour mettre en oeuvre une telle thérapie, les gènes intervenant à une étape précoce constituent une cible potentiellement intéressante. Tel est le cas des gènes *tat* et *rev* du virus VIH qui jouent un rôle essentiel dans l'initiation de la réplication virale.

Le gène *tat* code pour une protéine TAT trans-activatrice de l'expression de l'ensemble des gènes du VIH (Arya et al., 1985, Science, *229*, 69-73). Il semble que TAT intervienne aussi bien à un niveau transcriptionnel que post-transcriptionnel. Elle interagit spécifiquement avec une courte séquence nucléotidique, localisée à l'extrémité 5' du génome et des transcrits viraux et désignée séquence TAR (pour Trans-activation Responsive Region) (Rosen et al., 1985, Cell, *41*, 813-823).

La protéine REV, produit d'expression du gène *rev,* favorise le transport des ARN messagers (ARNm) de grande taille vers le cytoplasme, pour y être traduits. De son côté, REV reconnaît spécifiquement une séquence RRE (pour REV responsive element) sur les ARNm. Celle-ci est localisée à proximité d'une autre séquence dite CRS (*cis*-acting Repression Séquence), impliquée dans la rétention nucléaire des ARNm la portant. On suppose que la fixation de la protéine REV au niveau de sa séquence cible RRE a pour effet de contrebalancer l'effet inhibiteur de CRS sur le passage des grands ARNm viraux vers le cytoplasme.

Bien que leurs produits d'expression soient très différents d'un point de vue masse moléculaire, séquence et mécanisme d'action, des gènes régulateurs exerçant une fonction similaire à TAT et/ou REV ont été identifiés dans le génome d'autres virus infectieux, tels que les rétrovirus HTLV I et II (human T-cell leukemia virus) responsables de formes graves de leucémies et les virus de herpès (virus de la varicelle, du zona et d'Epstein Barr).

Depuis quelques années, des mutants négatifs et dominants (trans-dominants) de ces diverses protéines virales ont été décrits dans la littérature. En particulier, plusieurs équipes ont généré des variants trans-dominants des protéines TAT et REV capables de se fixer sur leur séquence cible mais incapables d'exercer la fonction de la protéine native. Si l'on a démontré leur effet inhibiteur vis à vis de l'action de la protéine native, il reste néanmoins à déterminer leur efficacité dans les cellules infectées par le VIH.

On a maintenant trouvé que l'expression dans une cellule infectée par le VIH, d'au moins deux gènes codant pour des variants trans-dominants, respectivement des protéines TAT et REV du virus VIH, permet de bloquer la propagation du virus. Les auteurs ont démontré que l'inhibition de l'infection virale résultant de l'expression conjointe des deux gènes est nettement supérieure à celle observée lors de l'expression de l'un quelconque de ces gènes. Cet effet synergique constitue un avantage inattendu.

Le but de la présente invention est de mettre à la disposition du public une composition pharmaceutique particulièrement efficace et donc, plus sûre en vue d'un usage humain, qui permette d'inhiber la réplication d'un virus à différents niveaux du cycle viral.

C'est pourquoi la présente invention a pour objet une composition comprenant au moins :
(a) un premier variant trans-dominant de la protéine TAT du virus VIH ; et
(b) un deuxième variant trans-dominant de la protéine REV du virus VIH, caractérisée en ce que le variant trans-dominant de la protéine REV a la séquence telle que montrée dans l'identificateur de séquence NO : 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine ; ci-après désigné REV (Gln⁷⁴, Leu⁷⁵ → Gly, Ser).

Dans le cadre de l'invention, un premier et un deuxième variant trans-dominant dérivent, du VIH. On regroupe sous la dénomination "VIH" les différentes souches et isolats viraux du virus agent étiologique de SIDA.

Par "mutant trans-dominant", on entend un mutant négatif et dominant c'est à dire un mutant non-fonctionnel (incapable d'exercer la fonction de la protéine native dont il dérive) mais capable d'inhiber de façon compétitive et dominante la fonction de cette dernière. En pratique, un variant trans-dominant peut être obtenu par délétion, substitution et/ou addition d'un ou plusieurs acides aminés de la protéine native ou d'un fragment fonctionnel de celle-ci. L'homme du métier connaît les techniques qui permettent d'effectuer ces modifications ainsi que les régions qui doivent être modifiées selon les protéines virales considérées.

Cependant et selon un mode avantageux, les premier et deuxième variants trans-dominants mis en oeuvre dans le cadre de la présente invention, dérivent de protéines virales intervenant à un stade précoce de l'infection et, notamment, de protéines capables d'interagir de manière spécifique avec une séquence nucléotidique déterminée (dite séquence cible) présente dans le génome et/ou les transcrits du virus dont elle sont issues.

On met en oeuvre un premier variant trans-dominant dérivé de la protéine, TAT du VIH (voir ci-après). A titre de deuxième variant trans-dominant, on emploiera un variant dérivé d'une protéine virale intervenant à une autre étape, de préférence précoce, de la réplication virale, par exemple au niveau du transport des ARNm viraux vers le cytoplasme de la cellule hôte.

Le choix d'un variant trans-dominant de la protéine TAT est très large. Il peut s'agir de ceux décrits dans l'art antérieur et notamment dans Green et al. (1989, Cell, *58*, 215-233) et Pearson et al. (1990, Proc. Natl. Acad. Sci. USA, *87*, 5079-5083). Mais il peut s'agir d'autres variants trans-dominants, notamment un variant ayant la séquence telle que montrée dans l'identificateur de séquence NO: 1, débutant à l'acide aminé + 1 et se terminant à l'acide aminé +86, dans laquelle :
(1) le résidu phénylalanine en position + 38 est remplacé par un résidu acide aspartique ;
(2) le résidu thréonine en position +40 est remplacé par un résidu alanine ;
(3) le résidu lysine en position +41 est remplacé par un résidu acide glutamique ;
(4) le résidu isoleucine en position +45 est remplacé par un résidu serine ; et/ou
(5) le résidu tyrosine en position +47 est remplacé par un résidu arginine.

Bien entendu, un variant trans-dominant TAT peut combiner plusieurs mutations.

A titre d'exemples de variants trans-dominants de la protéine REV, on peut citer ceux mentionnés dans Malim et al. (1989, Cell, *58*,205-214) et Venkatesh et Chinnadurai (1990, Virology, *178*, 327-330). D'autre part, l'emploi de variants modifiés au niveau de trois acides aminés et plus, en particulier au niveau des résidus Leu en position 75, 78 et 81 peut s'avérer avantageux. Pour illustrer ce mode de réalisation, on peut mentionner un variant REV muté en 5 positions dans lequel les codons codant pour les résidus Gln⁷⁴, Leu⁷⁵, Leu⁷⁸, Glu⁷⁹ et Leu⁸¹ ont été remplacés par des codons codant pour les résidus Gly, Ser, Glu, Phe et Asp respectivement.

La présente invention concerne également une composition selon l'invention, comme produit de combinaison pour une utilisation simultanée, séparée ou étalée dans le temps, pour le traitement ou la prévention des infections dues au virus dont sont issues les protéines virales en cause. L'usage d'une telle composition est plus particulièrement destinée à la thérapie anti-SIDA.

Conformément aux buts poursuivis par la présente invention, un premier et un deuxième variant trans-dominant peuvent être produits par voie recombinante dans une composition selon l'invention, à partir de vecteurs d'expression appropriés. On aura avantageusement recours à des vecteurs viraux dérivés des rétrovirus, adénovirus ou virus associé à l'adénovirus, dans lesquels on insère au moins une séquence d'ADN codant pour un variant trans-dominant en usage dans la présente invention. Le génome de ces vecteurs viraux est, de préférence, défectif pour la réplication. On indique que, dans le cadre de la présente invention, les vecteurs rétroviraux et notamment ceux dérivés du MoMuLV (Moloney murine leukemia virus) sont particulièrement préférés. De tels vecteurs ainsi que leurs techniques de préparation sont connus de l'homme de l'art.

Les séquences d'ADN codant pour un variant trans-dominant d'une protéine virale, peuvent être obtenues selon les techniques classiques de biologie moléculaire. Une des stratégies possibles consiste, tout d'abord, à isoler la séquence codant pour ladite protéine virale du génome viral, ceci par clonage ou PCR (polymerase chain reaction), puis de la modifier par mutagénèse dirigée, pour générer un variant trans-dominant désiré. Alternativement, on peut produire la séquence d'ADN d'intérêt par synthèse chimique.

Evidemment, les séquences d'ADN codant respectivement pour un premier et deuxième variant trans-dominant en usage dans la présente invention, peuvent être insérées dans un même vecteur recombinant ou dans deux vecteurs différents. De plus, ceux-ci peuvent comporter un gène de sélection facilitant l'isolement des clones transfectés par lesdits vecteurs.

La présente invention concerne également un vecteur recombinant comprenant:
(a) une première séquence d'ADN codant pour un variant trans-dominant de la protéine TAT du virus VIH, placée sous le contrôle des éléments nécessaires à son expression constitutive ; et
(b) une deuxième séquence d'ADN codant pour un variant trans-dominant de la protéine REV du virus VIH, ayant la séquence telle que montrée dans l'identificateur de séquence NO: 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine, placée sous le contrôle des éléments nécessaires à son expression constitutive.

Bien entendu, les première et deuxième séquences d'ADN sont placées sous le contrôle des éléments nécessaires à leur expression. Ces éléments comprennent notamment les éléments de régulation de la transcription d'une séquence d'ADN en ARNm ainsi que les signaux d'initiation et de terminaison de la traduction de l'ARNm en protéine. Parmi ces éléments, la région promotrice revêt une importance particulière.

D'une façon générale, on emploie une région promotrice fonctionnelle dans les cellules eucaryotes que l'on veut traiter et de préférence dans les cellules humaines. Une région promotrice peut être issue de gènes eucaryotes ou viraux, être constitutive ou régulable, notamment en réponse à certains signaux cellulaires tissu-spécifiques et, en particulier, lymphocyte-spécifiques. A titre d'exemples, on peut envisager d'employer les régions promotrices du virus SV40 (Simian Virus 40), du gène PGK (Phosphoglycérate Kinase), du gène HMG (Hydroxy Methyl-Glutaryl coenzyme A), du gène TK (Thymidine Kinase) du HSV-1, les LTRs (Long Terminal Repeat) du RSV (Rous Sarcoma Virus), du MoMuLV et du VIH et les promoteurs des gènes E1A, E3 et MLP (Major Late Promoter) de l'adénovirus. On indique que ces exemples ne sont pas limitatifs.

La présente invention s'étend aux cellules eucaryotes dans le génome desquelles sont insérées une première et une deuxième séquence mise en oeuvre dans le cadre de l'invention. Le ou les vecteurs portant lesdites séquences peuvent être introduits soit *in vitro* dans une cellule prélevée du patient, soit directement *in* *vivo*. De préférence, ladite cellule est une cellule humaine et de manière tout à fait préférée une cellule souche de la lignée hématopoïétique ou un lymphocyte.

L'invention s'adresse également à l'usage d'une composition, d'un vecteur ou d'une cellule selon l'invention pour la préparation d'un médicament destiné au traitement ou à la prévention des infections virales par thérapie génique et notamment des infections dues au VIH.

L'invention vise également une composition pharmaceutique caractérisée en ce qu'elle comprend à titre d'agent thérapeutique ou prophylactique une composition, un vecteur ou une cellule selon l'invention, en association avec un véhicule acceptable d'un point de vue pharmaceutique.

Une telle composition pharmaceutique selon l'invention, peut être préparée selon les techniques communément en usage et administrée selon toute voie d'administration connue. Par exemple, on associe l'agent thérapeutique ou prophylactique, en quantité thérapeutiquement efficace, à un support, un diluant ou un adjuvant acceptable. La quantité à administrer sera choisie en fonction de différents critères, en particulier de la voie d'administration choisie, de la pathologie concernée, des premières et deuxièmes séquences d'ADN à exprimer ou encore du patient et de la durée de traitement désirée.

Par ailleurs, l'invention a trait à un kit renfermant :
(a) un premier variant trans-dominant de la protéine TAT du virus VIH ; et
(b) un deuxième variant trans-dominant de la protéine REV du virus VIH, caractérisée en ce que le variant trans-dominant de la protéine REV a la séquence telle que montrée dans l'identificateur de séquence NO : 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine.

Enfin, l'invention concerne une méthode de traitement des infections virales et tout particulièrement dues au VIH, selon laquelle on administre une quantité thérapeutiquement efficace d'une composition, d'un vecteur ou d'une cellule selon l'invention à un patient ayant besoin d'un tel traitement.

A titre indicatif, les étapes essentielles pour un traitement anti-viral par thérapie génique, sont les suivantes :
- on effectue un prélèvement de moelle osseuse ou sanguin d'un patient ayant besoin d'un tel traitement ;
- on cultive les cellules prélevées (cellules souches ou lymphocytes) selon les techniques de l'art ;
- on transfecte un vecteur selon l'invention, dans lequel sont insérées les séquences codant pour un premier et un deuxième variant trans-dominant. Cependant, il est également possible de co-transfecter ou, alternativement, de transfecter de manière séparée et étalée dans le temps, chacun des vecteurs permettant l'expression de l'un ou l'autre des variants trans-dominants. On effectue éventuellement une étape supplémentaire de culture en milieu sélectif ; et
- on réinjecte les cellules ainsi modifiées dans le patient.

Naturellement, les modalités de ce protocole thérapeutique peuvent être sujettes à de nombreuses variantes déterminées par les cliniciens.

L'invention est illustrée ci-après par référence aux figures suivantes.

La Figure 1 schématise le vecteur pTG2350 permettant l'expression d'un variant trans-dominant TAT (Phe³⁸ → Asp) du VIH (TAT38), sous le contrôle du LTR 5' rétroviral.

La Figure 2 illustre le vecteur pTG2428 permettant l'expression du variant trans-dominant REV (Gln⁷⁴, Leu⁷⁵ → Gly, Ser) (REV*) du VIH sous le contrôle du LTR 5' rétroviral.

La Figure 3 montre les effets de l'expression conjointe des variants trans-dominants TAT et REV (◆) sur l'évolution de l'infection dans des cellules infectées par le VIH comparée à l'expression d'un variant trans-dominant TAT (■) ou celle d'un variant trans-dominant REV (□).

La Figure 4 schématise le vecteur rétroviral pTG4367 comprenant les séquences codant pour les variants trans-dominants TAT (Phe³⁸ → Asp) et REV (Gln⁷⁴, Leu⁷⁵, Leu⁷⁸, Glu⁷⁹ et Leu⁸¹ → Gly, Ser, Glu, Phe et Asp) indiqué ci-après REV**, sous le contrôle du promoteur PGK murin (PGKpro) et le LTR 5' rétroviral.

La Figure 5 illustre le vecteur pTG8301 dans lequel les séquences codant pour le mutant REV** et pour le produit de fusion TAT (Gln⁵⁴ → STOP) (TAT54) - néo sont placées sous le contrôle du LTR 5' rétroviral (LTR) et du promoteur PGK murin (PGK pro) respectivement.

La Figure 6 illustre le vecteur pTG8313 dans lequel les séquences codant pour le mutant REV** et pour le produit de fusion TAT (Phe³⁸ → Asp) - néo sont placées sous le contrôle du LTR 5' rétroviral (LTR) et du promoteur PGK murin (PGK pro) respectivement.

La Figure 7 illustre le vecteur pTG8315 dans lequel les séquences codant, d'une part, pour le mutant REV** et, d'autre part, pour les produits TAT38 et néo sont placées sous le contrôle du LTR 5' rétroviral (LTR) et du promoteur PGK murin (PGK pro) respectivement.

La Figure 8 illustre le vecteur pTG8323 dans lequel les séquences codant, d'une part, pour le mutant REV** et le produit Néo et, d'autre part, pour le mutant TAT (Gln⁵⁴ → STOP) sont placées sous le contrôle du mini LTR du VIH et du LTR 5' rétroviral respectivement.

### EXEMPLES

Les constructions décrites ci-après sont réalisées selon les techniques générales de génie génétique et de clonage moléculaire détaillées dans Maniatis et al. (1989, Laboratory Manual, Cold Spring Harbor Laboratory Press, Cold Spring Harbor, NY). L'ensemble des étapes de clonage mettant en oeuvre des plasmides bactériens est effectué par passage dans la souche *Escherichia coli* (*E*. *coli)* 1106, BJ5183 ou XL1-Blue, alors que celles mettant en oeuvre des vecteurs dérivés du phage M13 sont réalisées dans *E. coli* NM522.

En ce qui concerne les mutagénèses dirigées par oligodéoxynucléotides synthétiques, on applique le protocole décrit par Zoller et Smith (1982, Nucleic Acids Res., *10*, 6487) ou on met en oeuvre un kit d'origine commerciale selon les recommandations du fabricant. S'agissant de la réparation des sites de restriction, le remplissage des extrémités protubérantes peut être réalisé à l'aide du fragment Klenow de l'ADN polymérase d'*E*. *coli* et la destruction des extrémités 3' protubérantes en présence de l'ADN polymérase du phage T4 ou par traitement par la nucléase S1 suivi d'une réparation par la Klenow. Les techniques de PCR sont connues de l'homme de l'art et abondamment décrites dans PCR protocols, a guide to methods and applications (Ed : Innis, Gelfand, Sninsky et White, Academic Press Inc.).

D'autre part, la séquence d'un variant trans-dominant TAT ou REV est alignée sur celle de la protéine TAT ou REV du virus VIH-1, isolat Lai, telle que divulguée par Wain-Hobson et al. (1985, Cell, *40*, 9-17). En pratique, la numérotation des acides aminés dans la séquence d'un variant trans-dominant sera calquée sur celle établie pour la protéine native, séquence telle que montrée dans les identificateurs de séquence NO: 1 pour la protéine TAT et NO: 2 pour la protéine REV.

Par ailleurs, les lignées cellulaires sont cultivées selon les recommandations du fournisseur. D'une manière générale, les cellules sont transfectées selon la technique au phosphate de calcium (Maniatis et al., Molecular cloning : a laboratory manual, 1989, Cold Spring Harbor Laboratory), sauf les cellules CEM-A3 pour lesquelles on utilise l'électroporation. Cependant, d'autres protocoles permettant d'introduire un acide nucléique dans une cellule peuvent également être employés. Avant toute transfection, l'ADN est purifié sur gradient de chlorure de césium.

### EXEMPLE 1 : Etablissement d'une lignée cellulaire co-exprimant les variants trans-dominants TAT et REV.

### A. Construction du vecteur rétroviral pTG2350 pour l'expression du variant trans-dominant TAT (Phe ³⁸ → Asp) et caractérisation de son phénotype trans-dominant.

Un fragment d'ADN comportant l'ADN-copie (ADNc) correspondant aux 2 exons du gène *tat* du génome VIH-isolat Lai (Wain-Hobson et al. , 1985, Cell, *40*, 9-17 ; Sodroski et al., 1985, Science, *229*, 74-77) est modifié afin de créer un site *Bam*HI en 5' de l'ATG initiateur. En outre, un site *Bam*HI existe naturellement en 3' du codon stop. Le fragment *Bam*HI de 300pb comportant l'ADNc *tat* non muté est inséré dans le vecteur M13TG130 (Kieny et al., 1983, Gene, 26, 91-99). On obtient le vecteur M13TG2306.

On génère un variant trans-dominant TAT en remplaçant le résidu phénylalanine (Phe) en position 38 de la protéine TAT native par un acide aspartique (Asp). M13TG2306 est soumis à une mutagénèse dirigée en utilisant un kit commercial (Amersham, RPN 1523) et en mettant en oeuvre l'oligonucléotide décrit dans l'identificateur de séquence SEQ ID NO: 3, donnant M13TG2316.

Le fragment *Bam*HI isolé du vecteur précédent, est alors transféré dans le vecteur eucaryote pSG5 (Green et al., 1988, Nucleic Acids Res., *16,* 369). On obtient pTG2332, dans lequel l'expression du variant TAT (Phe³⁸ → Asp) est sous le contrôle du promoteur SV40.

On évalue la capacité du variant TAT (Phe³⁸ → Asp) à trans-activer l'expression du gène CAT (Chloramphenicol Acetyl Transferase) placé sous le contrôle du LTR du VIH incluant la séquence TAR (LTR-CAT ; Emerman et al., 1987, EMBO J., *6*, 37-55). L'activité trans-activatrice est déterminée par transfection transitoire en cellules HeLa (ATCC CCL2) (4x10⁵ cellules par boîte) de 1 µg du vecteur d'expression pTG2332 avec 0,5 µg du vecteur rapporteur LTR-CAT. On transfecte dans les mêmes conditions 1 µg de pHMG-Tat et 0,5 µg de LTR-CAT, qui constitue le témoin positif de trans-activation (100 %). pHMG-Tat est le vecteur d'expression de la protéine TAT native et résulte du clonage du fragment *Bam*HI de M13TG2306 dans le site *Bam*HI du vecteur pHMG (Mehtali et al., 1990, Gene, *91*, 179-184).

48h après les transfections, on mesure le niveau d'expression du gène CAT sur une aliquote d'extrait cellulaire correspondant à 10 à 20 µg en protéines selon la technique décrite dans Gorman et al. (1982, Mol. Cell. Biol., *2*, 1044-1051). La concentration en protéines est déterminée à l'aide d'un kit commercial en suivant le protocole indiqué par le fournisseur. Le pourcentage d'activité trans-activatrice du variant est calculé par rapport au taux mesuré avec le témoin positif (pHMG-Tat + LTR-CAT), celui-ci représentant 100 % d'activité trans-activatrice.

Puis, la capacité du variant TAT (Phe³⁸ → Asp) à inhiber la fonction trans-activatrice de la protéine TAT native, est déterminée par tranfection transitoire en cellules HeLa du vecteur d'expression pTG2332 (10 µg) avec le vecteur rapporteur LTR-CAT (0,5 µg) et le vecteur pHMG-Tat (1 µg). Comme précédemment, on mesure l'expression du gène rapporteur CAT sur une aliquote d'extrait correspondant à 10 à 20 µg en protéines. Le pourcentage d'inhibition de la protéine TAT native par le variant TAT (Phe³⁸ → Asp) est calculé par rapport au témoin résultant de la co-transfection de pHMG-Tat et de LTR-CAT qui représente 0 % d'activité inhibitrice.

Le variant TAT (Phe³⁸ → Asp) est un mutant trans-dominant efficace puisque son activité trans-activatrice est réduite (expression du gène CAT inférieure à 10 % par rapport à celle mesurée avec la protéine TAT native) et puisqu'il inhibe fortement l'activité de la protéine TAT native (au moins 90 % d'inhibition).

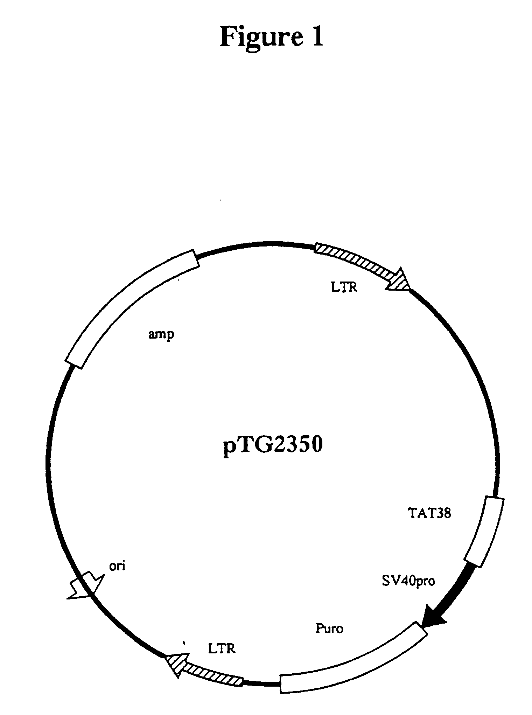

Le fragment *Bam*HI de 300pb isolé de M13TG2316, est inséré dans le site *Bam*HI du vecteur rétroviral pLXSP, générant pTG2350 (Figure 1). Le vecteur pLXSP dérive du pLXSN (Miller et Rossman, 1989, Biotechniques, *7,* 980-988) après remplacement du gène néomycine (*néo*) par un gène conférant une résistance à la puromycine et du LTR 3' MSV (Myelo Sarcoma Virus) par le LTR 3' MPSV (Myelo Proliferative Sarcoma Virus).

### B. Construction des vecteurs rétroviraux pTG2428 et pTG2430 pour l'expression du variant trans-dominant REV (Gln⁷⁴, Leu⁷⁵ → Gly, Ser) et caractérisation de son phénotype trans-dominant.

Un fragment *Sal*I de 830 pb comprenant l'ADN-copie correspondant aux deux exons codants du gène *rev* du VIH1-isolat Lai est inséré dans le vecteur M13TG130, générant le vecteur M13TG2309. Celui-ci est modifié par mutagénèse dirigée (kit Amersham) afin d'introduire deux sites *Bgl*II encadrant la séquence codant pour REV, le premier en amont de l'ATG initiateur et le second en aval du codon stop. La présence de ces sites *Bgl*II permet de faciliter les étapes de clonage ultérieures. Mais, tout autre site de restriction adéquate aurait pu être choisi.

Le vecteur ainsi obtenu est soumis à une mutagénèse dirigée en mettant en oeuvre l'oligonucléotide OTG4672 reporté dans l'identificateur de séquence NO: 4, dans le but de substituer les résidus Gln et Leu en position 74 et 75 de la protéine REV native par les résidus Gly et Ser. Le vecteur en résultant est digéré par *Bgl*II et le fragment de 390 pb est inséré dans le vecteur d'expression eucaryotique pSG5 pour donner le plasmide pTG REV.TD, sur lequel on évalue le phénotype trans-dominant du mutant REV (Gln⁷⁴, Leu⁷⁵ → Gly, Ser).

Son activité sur le transport des ARNm est déterminée par transfection transitoire dans la lignée cellulaire HeLa de pTG REV.TD (1 µg) et du vecteur rapporteur pTG1335 (0,5 µg). Ce dernier comprend le gène CAT placé sous le contrôle du promoteur SV40 et les éléments RRE/CRS du génome VIH1 insérés en aval du gène CAT. Ainsi, en l'absence d'une protéine REV fonctionnelle, l'élément CRS va maintenir les ARNm dans le noyau et on ne détecte pas de produit d'expression du gène CAT. Par contre, en présence d'une protéine REV fonctionnelle, celle-ci va se fixer sur sa séquence cible RRE et induire le transport des ARNm vers le cytoplasme et leur traduction.

On détermine l'expression du gène CAT sur une aliquote d'extrait cellulaire dans les mêmes conditions que précédemment. Le pourcentage d'activité du mutant REV (Gln⁷⁴ Leu⁷⁵ → Gly, Ser) est calculé par rapport au taux mesuré avec un témoin positif résultant de la co-transfection du vecteur pHMG-Rev permettant l'expression de la protéine REV native et du vecteur rapporteur pTG1335, représentant 100 % d'activité REV.

Parallèlement, on évalue sa capacité à inhiber la fonction de la protéine REV native, par transfection transitoire du vecteur d'expression pTG REV.TD (10 µg) et du vecteur rapporteur pTG1335 (0,5 µg) en présence de 1 µg du vecteur pHMG-Rev. Le taux d'expression du gène CAT est mesuré par rapport au témoin résultant de la co-transfection de pHMG-Rev et de pTG1335 et qui représente 0 % d'inhibition.

Le variant REV (Gln⁷⁴, Leu⁷⁵ → Gly, Ser) s'avère être un variant trans-dominant efficace qui n'a pratiquement plus d'activité dans le transport des ARNm vers le cytoplasme mais qui est capable d'inhiber de façon dominante la fonction REV native.

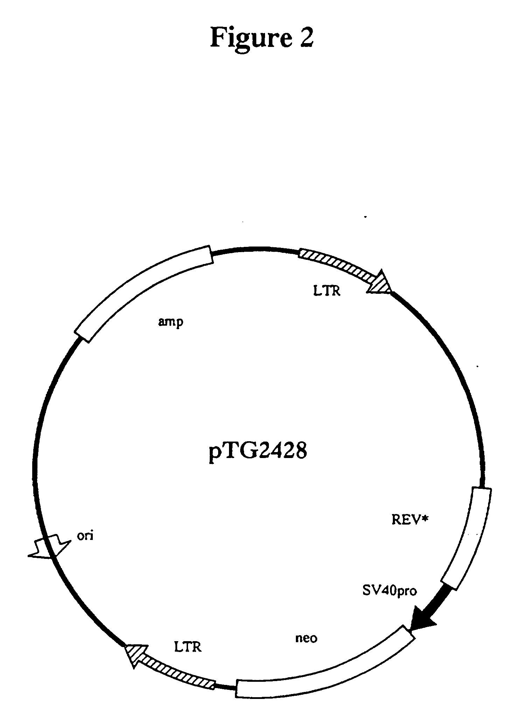

Le fragment *Bgl*II de 390 pb portant la séquence codante pour le mutant trans-dominant REV est sous cloné dans le site *Bam*HI de pTG5192. Ce dernier dérive de pLXSN, dans lequel le LTR 3' issu du MSV est remplacé par le LTR 3' du MPSV. On génère le vecteur rétroviral pTG2428 (Figure 2) portant également le marqueur de sélection néomycine qui confère la résistance au G418.

D'autre part, ce même fragment *Bgl*II est également inséré dans le vecteur rétroviral pLXSP, pour donner pTG2430 porteur du gène de résistance à la puromycine.

### C. Etablissement d'une lignée cellulaire CEM-A3 exprimant les mutants trans-dominants TAT (Phe³⁸ → Asp) et REV (Gln⁷⁴, Leu⁷⁵ → Gly, Ser).

On établit une lignée cellulaire stable co-produisant les deux variants trans-dominants à partir des cellules naturellement infectables par le VIH, par exemple la lignée lymphocytaire humaine CEM-A3 dérivée des CCRF-CEM (ATCC CCL119) et qui exprime fortement le marqueur de surface CD4, récepteur du VIH.

On électropore 20 µg d'ADN plasmidique pTG2350 dans 2x10⁷ cellules CEM-A3 (Gene pulser Biorad, voltage 210V, capacitance 960µF). Puis les cellules sont remises en culture dans du milieu RPMI (Gibco-BRL) à 37°C en présence de 5 % de CO₂. Deux jours plus tard, on place les cellules en milieu sélectif (puromycine 0,5 µg/ml) et on isole les clones résistants (cellules CEM-A3 2350).

On peut détecter l'expression du variant trans-dominant dans les différents clones par transfection transitoire par les vecteurs LTR-CAT et pHMG-Tat et en mesurant l'extinction de l'expression du gène CAT par rapport à un témoin de cellules d'origine CEM-A3 transfectées avec ces deux vecteurs.

On sélectionne un clone résistant à la puromycine, dans lequel on introduit 20 µg de pTG2428 selon le protocole d'électroporation indiqué ci-dessus. On place les cellules en milieu doublement sélectif (puromycine 0,5 µg/ml et G418 1 mg/ml). Le pool de cellules résistantes aux deux antibiotiques est désigné ci-après CEM-A3 2350 + 2428.

A titre de comparaison, on établit également une lignée CEM-A3 productrice du variant trans-dominant REV par transfection du vecteur pTG2430. Les cellules résistantes à la puromycine sont désignées CEM-A3 2430.

### EXEMPLE 2 : Evaluation de la résistance des cellules CEM-A3 2350+2428 à une infection par le VIH.

On centrifuge à basse vitesse, environ 10⁶ cellules CEM-A3 2350+2428. Après lavage, le culot cellulaire est repris dans 200 µl de milieu RPMI en absence de sérum. Les cellules sont infectées par une préparation de VIH à une TCID₅₀ comprise entre 1 et 1000, et de préférence 50 à 100. La TCID₅₀ est déterminée selon la méthode décrite dans Reed et al. (1938, Am. J. Hyg., *27,* 493-497), sur lignées cellulaires infectables par le VIH, comme par exemple les cellules CEM-A3, et correspond à la dose à laquelle 50 % des cellules sont infectées et 50 % ne le sont pas. Elle est vérifiée pour chaque lot de VIH employé. L'infection est poursuivie 1h à 37° C.

Les cellules sont ensuite lavées 2 fois dans du RPMI et mises en culture dans 5 ml de ce même milieu, complémenté avec 10 % de SVF (sérum de veau foetal). Cette étape constitue le T=0 de l'expérimentation. Le milieu est renouvelé 2 ou 3 fois par semaine. La propagation du virus est évaluée par mesure de l'activité réverse-transcriptase à intervalles réguliers dans le surnageant de culture selon la méthode décrite dans Spire et al. (1985, Lancet i, 188-189).

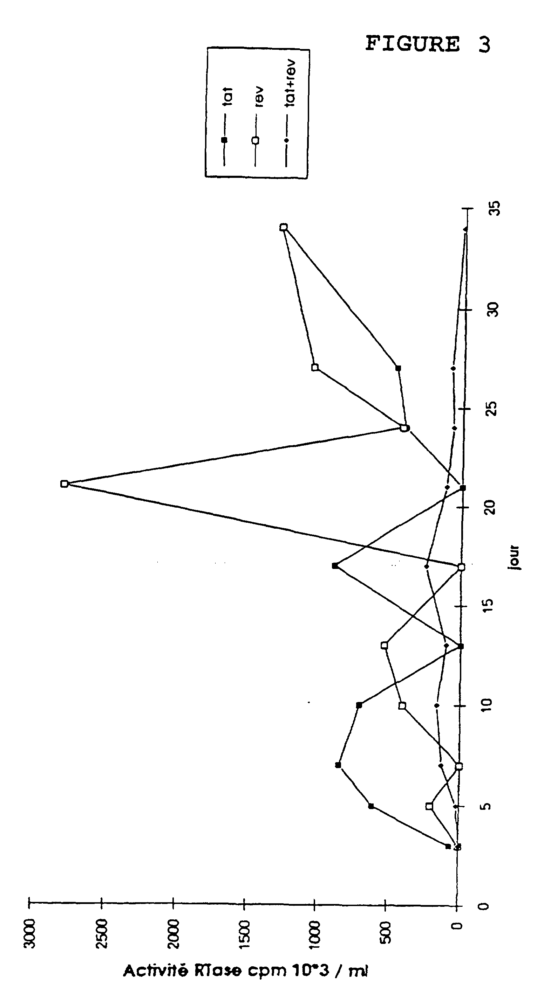

Les résultats illustrés dans la Figure 3, montrent que l'activité réverse-transcriptase des surnageants CEM-A3 2350 + 2428 se situe à un niveau très bas pendant au moins 35 jours de culture. On indique que dans les cellules normales infectées par le VIH, on détecte normalement une activité réverse-transcriptase au bout de 5 à 6 jours, du fait du relargage de nouvelles particules virales dans le milieu, qui va en augmentant rapidement au cours du temps jusqu'à la mort cellulaire.

A titre comparatif, on effectue l'expérimentation sur des aliquotes de cultures cellulaires CEM-A3 2350 et CEM-A3 2430 dans des conditions strictement identiques. Dans ce cas on observe un retard dans la propagation du virus se traduisant par une activité réverse-transcriptase réduite pendant les 20 premiers jours de culture, mais suivie d'une augmentation lorsque la culture est poursuivie.

### EXEMPLE 3 : Construction de vecteurs rétroviraux co-exprimant des variants trans-dominants TAT et REV et établissement de lignées amphotropes correspondantes.

### A. Construction du vecteur pTG4367

Le vecteur M13TG2309 est soumis à une mutagénèse dirigée à l'aide de l'oligonucléotide OTG5254 reporté dans l'identificateur de séquence NO: 5, qui permet de modifier la séquence d'ADN codant pour la protéine REV native en 5 endroits pour créer un variant trans-dominant REV (remplacement des codons codant pour les résidus Gln⁷⁴ en Gly, Leu⁷⁵ en Ser, Leu⁷⁸ en Glu, Glu⁷⁹ en Phe et Leu⁸¹ en Asp). On obtient le vecteur M13TG4303.

Le vecteur rétroviral pLXSP est digéré par *Eco*RI-*Hpa*I. On isole un fragment *Eco*RI-*Pst*I portant le promoteur PGK murin décrit dans Adra et al. (1987, Gene, *60*, 65-74), le site *Pst*I étant rendu franc par traitement à la T4 polymérase. Le vecteur ainsi obtenu est délété de la cassette d'expression du gène de résistance à la puromycine. Pour ce faire, après digestion par *Cla*I-*Bam*HI et traitement par le fragment Klenow de l'ADN polymérase, il est religué sur lui-même afin de générer pTG2673.

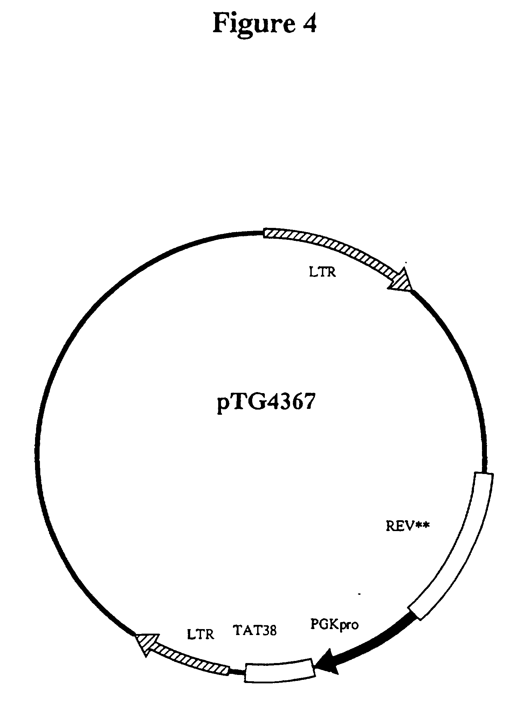

On introduit le fragment *Bam*HI isolé de M13TG2316 dans le vecteur pTG2673 préalablement digéré par *Bam*HI. Puis, le vecteur obtenu est digéré par *Eco*RI et traité par le fragment Klenow de l'ADN polymérase. En parallèle, M13TG4303 est digéré par *Bgl*II, traité par la Klenow puis digéré par *Pvu*II. On isole le fragment porteur de la séquence codant pour le variant trans-dominant REV 5 fois muté, que l'on insère dans pTG2673 ainsi traité. On obtient pTG4367 (figure 4) dans lequel la séquence d'ADN codant pour TAT (Phe³⁸ → Asp) est placée sous le contrôle du promoteur PGK et celle codant pour REV trans-dominant sous le contrôle du LTR 5' du vecteur rétroviral.

On établit une lignée amphotrope à partir d'une lignée cellulaire, dite d'encapsidation, dans laquelle est inséré un gène *env* d'un rétrovirus amphotrope, par exemple la lignée PA317 (Miller et al., 1986, Mol. Cell. Biol., *6,* 2895-2902), ou Gp. Env. Am. 12 (Markowitz et al., 1988, Virology *167*, 400-406). On transfecte le vecteur pTG4367 et un vecteur de sélection quelconque, comme par exemple un vecteur comportant le gène de résistance à la puromycine sous le contrôle du promoteur SV40. Le jour suivant la transfection, les cellules sont placées en milieu sélectif (puromycine 6 µg/ml). On isole les cellules résistantes, productrices de particules virales amphotropes capables d'infecter les cellules humaines.

On peut constituer un stock viral qui pourrait être utilisé en thérapie génique pour l'infection des cellules souches hématopoïétiques ou des lymphocytes d'un patient.

### B. Construction du vecteur pTG8301 (expression de TAT (Gln⁵⁴ → STOP) fusionné au produit du gène néo et de REV muté au niveau des résidus 74, 75, 78, 79 et 81)

La construction du variant trans-dominant TAT dans lequel le résidu glutamine (Gln) en position 54 de la protéine native est remplacé par un codon stop, est décrite dans la demande européenne EP 0614 980. Pour résumer, le vecteur M13TG2306 (Exemple 1) est soumis à une mutagénèse dirigée (kit Amersham) à l'aide de l'oligonucléotide OTG4376 (voir SEQ ID NO: 3 de EP 0614 980). On obtient M13TG2330. Afin de réaliser la fusion avec le gène de sélection, celui-ci est à nouveau soumis à une mutagénèse dirigée dans le but de détruire le codon STOP du gène *tat* et d'y insérer un bras de trois résidus alanine correspondant à un site *Not*I. On utilise l'oligonucléotide mutagène OTG4413 (SEQ ID NO: 6) et on génère M13TG2330*.

En parallèle, le gène de sélection *néo* est amplifié par PCR à partir du vecteur pAG60 (Colbère-Garapin et al., 1981, J. Mol. Biol., *150*, 1-14). Pour ce faire, on met en oeuvre des amorces conventionnelles munies à leurs extrémités 5' de sites *Not*I. Le fragment PCR ainsi obtenu est cloné dans M13TG2330* linéarisé par *Not*I pour donner M13TG6389. Après digestion de ce dernier par *Bam*HI, on purifie le fragment de 1,2 kb portant le gène de fusion codant pour le produit hybride TAT suivi de néo. Celui-ci est introduit dans le site *Bam*HI du vecteur rétroviral pTG2673 pour donner pTG6392.

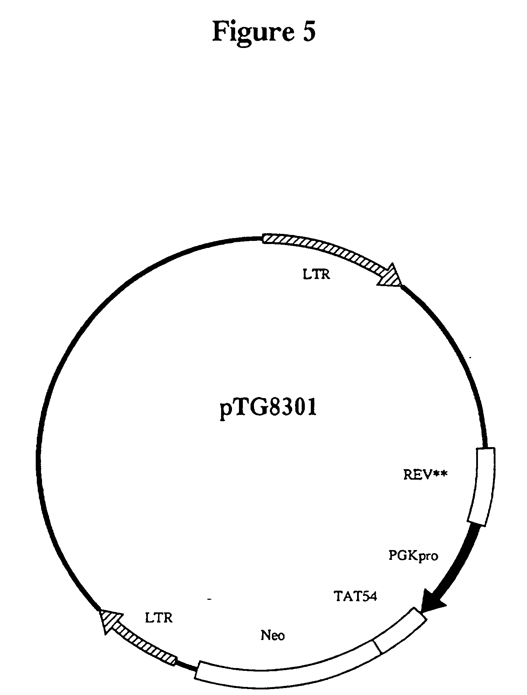

Le vecteur M13TG4303 est soumis à une mutagénèse dirigée afin de créer un site *Bgl*II en aval du codon stop du trans-dominant REV, dans le but de faciliter les étapes de clonage ultérieures. On emploi à cet effet l'oligonucléotide OTG 4370 (SEQ ID NO: 7). Puis, le fragment *Bgl*II (0,38 kb) ainsi généré est finalement inséré dans le vecteur pTG6392. On obtient pTG8301 (Figure 5) dans lequel le gène codant pour le mutant REV trans-dominant est placé sous le contrôle du LTR 5' du vecteur rétroviral alors que l'expression du variant TAT trans-dominant fusionné au produit du gène *néo* dépend du promoteur PGK murin.

### C. Construction du vecteur pTG8313 (expression de TAT (Phe³⁸ → Asp) fusionné au produit du gène néo et de REV muté au niveau des résidus 74, 75, 78, 79 et 81)

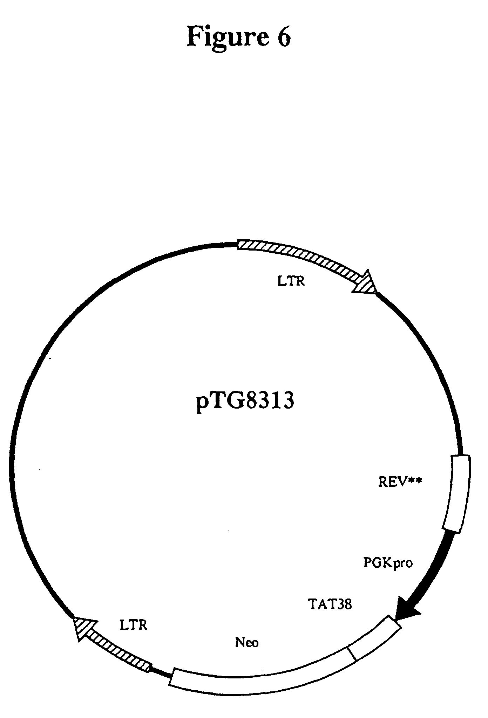

Le vecteur M13TG6390 résulte de l'insertion du fragment *Not*I de M13TG6389 portant le gène *néo* dans le M13TG2316. Au préalable, un site *Not*I a été créé dans ce dernier en amont du codon STOP du mutant TAT38 à l'aide de l'oligonucléotide mutagène OTG4413 décrit précédemment. Puis, le fragment *Bam*HI (1,2 kb) purifié de M13TG6390 est introduit dans le vecteur pTG2673 préalablement digéré par *Bam*HI pour donner pTG8305. Enfin, le fragment *Bgl*II de 0,38 kb comprenant le gène rev muté est cloné dans le site *Eco*RI (traité à la Klenow) du vecteur obtenu à l'étape précédente pour générer pTG8313 (Figure 6).

### D. Construction des vecteurs rétroviraux pTG8315 et pTG8316

Le fragment *Bam*HI de 0,3 kb issu de M13TG2316 (portant TAT Phe³⁸ → Asp) ou de M13TG2330 (TAT (Gln⁵⁴ → STOP) est inséré dans le site *Bam*HI du p polyIII-I* (Lathe et al., Gene, 1987, *57*, 193-201), donnant lieu à p poly-2316 ou p poly-2330. Ceux-ci sont clivés par *Eco*RI et *Pvu*II et on introduit un fragment *Eco*RI-*Nco*I (dont les sites sont rendus francs par traitement par la Klenow) correspondant aux éléments IRES du virus EMCV (Eucephalomyocarditis Virus). Celui-ci dérive du vecteur IRES-βgeo décrit dans la demande internationale WO 94/24301.

Puis le gène néo est cloné en aval de l'IRES sous forme d'un fragment à extrémités franches isolé de pAG60. L'homme du métier sait comment générer un fragment franc à partir d'un plasmide de l'art antérieur. Il est introduit dans le site *Acc*I (traité par la Klenow) de la construction précédente. On obtient les vecteurs pTG6399 et pTG6398 comprenant respectivement les cassettes bicistroniques TAT (Phe³⁸ → Asp) - IRES - *néo* et TAT (Gln⁵⁴ → STOP) - IRES - *néo*.

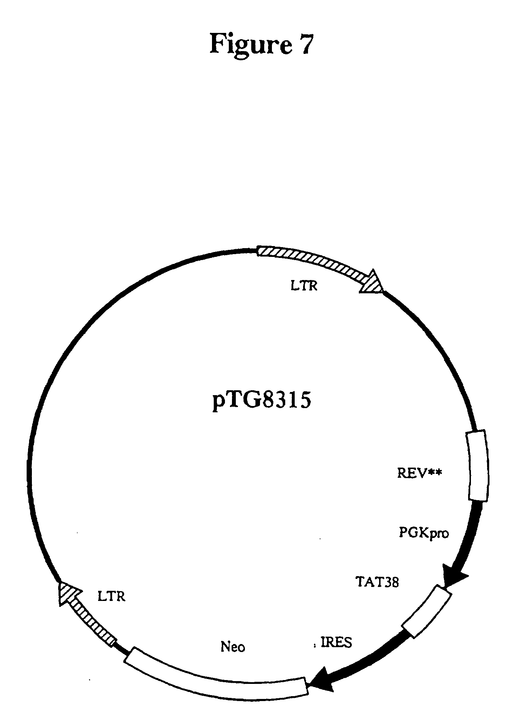

En parallèle, le fragment *Bgl*II (0,38 kb) purifié de M13TG4303 muté est traité par la Klenow puis inséré dans le vecteur pTG2673 digéré par *Eco*RI et également traité par la Klenow. Le vecteur ainsi obtenu est finalement digéré par *Xho*I afin de cloner le fragment *Xho*I obtenu de pTG6399 ou de pTG6398. On génère ainsi les vecteurs pTG8315 (Figure 7) et pTG8316 dans lesquels le gène rev modifié (5 mutations) est sous le contrôle du LTR 5' rétroviral et la cassette bicistronique codant pour TAT (Phe³⁸ → Asp) ou TAT (Gln⁵⁴ → STOP) et le produit du gène *néo*, est sous le contrôle du promoteur PGK.

### E. Construction des vecteurs inductibles par la protéine TAT native du VIH

Le fragment *Bgl*II (0,38 kb) portant le gène *rev* trans-dominant est purifié de M13TG4303 muté et sous-cloné dans le site *Bam*HI de p polyIII-I*. Le vecteur en résultant est linéarisé par *Sma*I et on y introduit le fragment *Eco*RI°*-Nco*I° portant l'IRES. Puis, on insère dans le site *Eco*RV du vecteur ainsi généré le gène *néo* sous forme d'un fragment à extrémités franches décrit ci-dessus, de manière à générer une cassette bicistronique contenant les séquences codant pour REV (Gln⁷⁴, Leu⁷⁵, Leu⁷⁸, Glu⁷⁹, Leu⁸¹ → Gly, Ser, Glu, Phe, Asp) et le produit du gène néo dont la traduction est réinitiée par l'IRES EMCV.

Cette cassette est isolée par digestion *Xho*I-*Xba*I suivie d'un traitement par la Klenow et insérée dans le site *Bal*I du vecteur pTG4347. Ce dernier résulte du clonage dans le p polyIII-I* d'un fragment comportant la partie 3' du LTR 5' du VIH (isolat Lai), ci-après désigné "mini-LTR", et le signal de polyadénylation du virus SV40. A titre indicatif, le mini LTR correspond au fragment *Sca*I-*Hind*III du LTR 5' VIH et possède les éléments assurant la transcription et la trans-activation par la protéine TAT native. Le vecteur ainsi généré est nommé pTG8314.

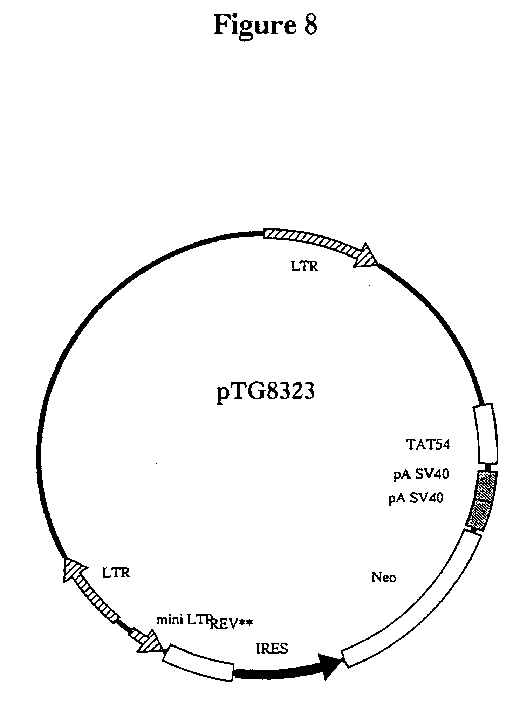

En parallèle, les fragments *Bam*HI de M13TG2316 et M13TG2330 comprenant les séquences codant pour les variants trans-dominants TAT (Phe³⁸ → Asp) et TAT (Gln⁵⁴ → STOP) respectivement, sont insérés dans le vecteur pTG2682, lequel correspond à pTG2673 dépourvu du promoteurs PGK. Puis, le fragment *Not*I isolé de pTG8314, traité par la Klenow est finalement introduit dans le site *Xho*I (rendu franc par traitement à la Klenow) de l'une et l'autre des constructions précédentes issues de pTG2682. On obtient des vecteurs dans lesquels les gènes *tat* trans-dominants respectifs sont exprimés de façon constitutive sous le contrôle du LTR 5' rétroviral tandis que le gène *rev* trans-dominant est exprimé de façon inductible par la protéine TAT native du VIH sous contrôle du mini LTR du VIH. On selectionne de préférence des clones dans lesquels les deux cassettes d'expression sont en orientation inverse l'une par rapport à l'autre (Figure 8 ; pTG8323).

### F. Evaluation de la fonctionnalité des constructions précédentes

L'activité trans-dominante des produits TAT et REV produit par chacune des constructions de l'Exemple 3 peut être évaluée comme précédemment, par co-transfection transitoire de cellules humaines (par exemple de cellules A549 ATCC CCL185) avec un vecteur rapporteur (LTR-CAT dépendant de TAT ou pTG1335 dépendant de REV) et un vecteur d'expression de la protéine TAT ou REV sauvages (pHMG-Tat ou pHMG-Rev).

### G. Constitution de particules virales infectieuses

On peut préparer des stocks de particules virales infectieuses comprenant chacune des constructions de l'exemple 3 de façon conventionnelle. Brièvement, les vecteurs rétroviraux sont transfectés dans les cellules d'encapsidation écotropes GP+E 86 (Markowitz et al., 1988, J. Virol., *62*, 1120-1124). Après sélection des cellules résistantes à la néomycine, les surnageants de culture sont récoltés pour transduire une lignée d'encapsidation amphotrope, par exemple la lignée PA317. Les lignées ainsi obtenues constitueront les cellules productrices des particules virales infectieuses, lesquelles peuvent être utilisées dans le cadre d'une thérapie génique anti-SIDA. Leur capacité à induire une résistance à l'infection par le VIH peut être évaluée *in vivo* et *in vitro* comme indiqué à l'exemple 2.

## Revendications

1. Une composition comprenant au moins :
(a) un premier variant trans-dominant de la protéine TAT du virus VIH ; et
(b) un deuxième variant trans-dominant de la protéine REV du virus VIH, **caractérisée en ce que** le variant trans-dominant de la protéine REV a la séquence telle que montrée dans l'identificateur de séquence NO : 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine.

2. Une composition selon la revendication 1, **caractérisée en ce que** le variant trans-dominant de la protéine REV a en outre le résidu leucine en position +78 remplacé par un résidu acide glutamique, le résidu acide glutamique en position +79 remplacé par un résidu phenylalanine, et le résidu leucine en position +81 remplacé par un résidu acide aspartique.

3. Une composition selon la revendication 1 ou 2, **caractérisée en ce que** le variant trans-dominant de la protéine TAT a la séquence telle que montrée dans l'identificateur de séquence NO: 1 débutant à l'acide aminé +1 et se terminant à l'acide aminé +86 dans laquelle :
(1) le résidu phénylalanine en position +38 est remplacé par un résidu acide aspartique ;
(2) le résidu thréonine en position +40 est remplacé par un résidu alanine ;
(3) le résidu lysine en position +41 est remplacé par un résidu acide glutamique ;
(4) le résidu isoleucine en position +45 est remplacé par un résidu serine ; et/ou
(5) le résidu tyrosine en position +47 est remplacé par un résidu arginine.

4. Une composition selon l'une des revendications 1 à 3, comme produit de combinaison pour une utilisation simultanée, séparée ou étalée dans le temps destinée au traitement ou à la prévention des infections dues au VIH.

5. Un vecteur recombinant comprenant :
(a) une première séquence d'ADN codant pour un variant trans-dominant de la protéine TAT du virus VIH, placée sous le contrôle des éléments nécessaires à son expression constitutive ; et
(b) une deuxième séquence d'ADN codant pour un variant trans-dominant de la protéine REV du virus VIH, ayant la séquence telle que montrée dans l'identificateur de séquence NO : 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine, placée sous le contrôle des éléments nécessaires à son expression constitutive

6. Un vecteur recombinant selon la revendication 5, **caractérisé en ce que** le variant trans-dominant de la protéine REV a en outre le résidu leucine en position +78 remplacé par un résidu acide glutamique, le résidu acide glutamique en position +79 remplacé par un résidu phenylalanine, et le résidu leucine en position +81 remplacé par un résidu acide aspartique.

7. Un vecteur recombinant selon l'une des revendications 5 et 6, **caractérisé en ce que** le variant trans-dominant de la protéine TAT a la séquence telle que montrée dans l'identificateur de séquence NO : 1 débutant à l'acide aminé +1 et se terminant à l'acide aminé +86 dans laquelle :
(1) le résidu phénylalanine en position +38 est remplacé par un résidu acide aspartique ;
(2) le résidu thréonine en position +40 est remplacé par un résidu alanine ;
(3) le résidu lysine en position +41 est remplacé par un résidu acide glutamique ;
(4) le résidu isoleucine en position +45 est remplacé par un résidu serine ; et/ou
(5) le résidu tyrosine en position +47 est remplacé par un résidu arginine.

8. Un vecteur recombinant selon l'une des revendications 5 à 7, **caractérisé en ce que** les éléments nécessaire à l'expression constitutive sont le promoteur PGK ou le LTR du virus MoMuLV.

9. Un vecteur recombinant selon l'une des revendications 5 à 8, **caractérisé en ce qu'**il dérive d'un virus sélectionné parmi les rétrovirus, adénovirus et virus associé à l'adénovirus.

10. Une cellule eucaryote dans le génome de laquelle sont insérées :
(a) une première séquence d'ADN codant pour un variant trans-dominant de la protéine TAT du virus VIH, placée sous le contrôle des éléments nécessaires à son expression constitutive ; et
(b) une deuxième séquence d'ADN codant pour un variant trans-dominant de la protéine REV du virus VIH, ayant la séquence telle que montrée dans l'identificateur de séquence NO : 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine, placée sous le contrôle des éléments nécessaires à son expression constitutive.

11. Une cellule eucaryote selon la revendication 10, **caractérisée en ce que** le variant trans-dominant de la protéine REV a en outre le résidu leucine en position +78 remplacé par un résidu acide glutamique, le résidu acide glutamique en position +79 remplacé par un résidu phenylalanine, et le résidu leucine en position +81 remplacé par un résidu acide aspartique.

12. Une cellule eucaryote selon l'une des revendications 10 à 11, **caractérisée en ce que** le variant trans-dominant de la protéine TAT a la séquence telle que montrée dans l'identificateur de séquence NO : 1 débutant à l'acide aminé +1 et se terminant à l'acide aminé +86 dans laquelle :
(1) le résidu phénylalanine en position +38 est remplacé par un résidu acide aspartique ;
(2) le résidu thréonine en position +40 est remplacé par un résidu alanine ;
(3) le résidu lysine en position +41 est remplacé par un résidu acide glutamique ;
(4) le résidu isoleucine en position +45 est remplacé par un résidu serine ; et/ou
(5) le résidu tyrosine en position +47 est remplacé par un résidu arginine.

13. Une cellule eucaryote selon l'une des revendications 10 à 12, **caractérisé en ce que** les éléments nécessaires à l'expression constitutive sont le promoteur PGK ou le LTR du virus MoMuLV.

14. Usage d'un vecteur recombinant selon l'une des revendications 5 à 9 ou d'une cellule selon l'une des revendications 10 à 13 pour la préparation d'un médicament destiné au traitement ou à la prévention des infections virales dues au VIH.

15. Une composition selon l'une des revendications 1 à 3 qui comprend en outre un véhicule acceptable d'un point de vue pharmaceutique.

16. Une composition pharmaceutique comprenant un vecteur recombinant selon l'une des revendications 5 à 9 ou une cellule selon l'une des revendications 10 à 13 en association avec un véhicule acceptable d'un point de vue pharmaceutique.

17. Un kit comprenant
(a) un premier variant trans-dominant d'une protéine TAT du virus VIH, et
(b) un deuxième variant trans-dominant de la protéine REV du virus VIH, **caractérisé en ce que** le variant trans-dominant de la protéine REV a la séquence telle que montrée dans l'identificateur de séquence NO : 2 débutant à l'acide aminé +1 et se terminant à l'acide aminé +116 dans laquelle le résidu glutamine en position +74 est remplacé par un résidu glycine et le résidu leucine en position +75 est remplacé par un résidu sérine ;
avec des instructions pour leur administration concomitante ou séquentielle.

## Patentansprüche

1. Zusammensetzung, mindestens umfassend:
(a) eine erste transdominante Variante des TAT-Proteins des HIV-Virus; und
(b) eine zweite transdominante Variante des REV-Proteins des HIV-Virus, **dadurch gekennzeichnet, dass** die transdominante Variante des REV-Proteins die Sequenz aufweist, wie sie in der Identifizierung der Sequenz NO: 2 beginnend mit der Aminosäure +1 und endend bei der Aminosäure +116 gezeigt ist, in der der Glutaminrest in der Position +74 durch einen Glycinrest ersetzt ist und der Leucinrest in der Position +75 durch einen Serinrest ersetzt ist.

2. Zusammensetzung nach Anspruch 1, **dadurch gekennzeichnet, dass** bei der transdominanten Variante des REV-Proteins darüber hinaus der Leucinrest in der Position +78 durch einen Glutaminsäurerest ersetzt ist, der Glutaminsäurerest in der Position +79 durch einen Phenylalaninrest ersetzt ist und der Leucinrest in der Position +81 durch einen Asparaginsäurerest ersetzt ist.

3. Zusammensetzung nach Anspruch 1 oder 2, **dadurch gekennzeichnet, dass** die transdominante Variante des TAT-Proteins die Sequenz aufweist, wie sie in der Identifizierung der Sequenz NO: 1 beginnend mit der Aminosäure +1 und endend bei der Aminosäure +86 gezeigt ist, in der:
(1) der Phenylalaninrest in der Position +38 durch einen Asparaginsäurerest ersetzt ist;
(2) der Threoninrest in der Position +40 durch einen Alaninrest ersetzt ist;
(3) der Lysinrest in der Position +41 durch einen Glutaminsäurerest ersetzt ist;
(4) der Isoleucinrest in der Position +45 durch einen Serinrest ersetzt ist; und/oder
(5) der Tyrosinrest in der Position +47 durch einen Argininrest ersetzt ist.

4. Zusammensetzung nach einem der Ansprüche 1 bis 3 als Kombinationsprodukt für eine gleichzeitige, getrennte oder über die Zeit verteilte Verwendung, die zur Behandlung oder Verhütung von auf HIV zurückzuführenden Infektionen bestimmt ist.

5. Rekombinanter Vektor, umfassend:
(a) eine erste DNA-Sequenz, die eine transdominante Variante des TAT-Proteins des HIV-Virus kodiert und unter die Kontrolle von Elementen gestellt ist, die für ihre konstitutive Expression erforderlich sind;
(b) eine zweite DNA-Sequenz, die eine transdominante Variante des REV-Proteins des HIV-Virus kodiert und die Sequenz aufweist, wie sie in der Identifizierung der Sequenz NO: 2 beginnend mit der Aminosäure +1 und endend bei der Aminosäure +116 gezeigt ist, in der der Glutaminrest in der Position +74 durch einen Glycinrest ersetzt ist und der Leucinrest in der Position +75 durch einen Serinrest ersetzt ist und die unter die Kontrolle von Elementen gestellt ist, die für ihre konstitutive Expression erforderlich sind.

6. Rekombinanter Vektor nach Anspruch 5, **dadurch gekennzeichnet, dass** bei der transdominanten Variante des REV-Proteins darüber hinaus der Leucinrest in der Position +78 durch einen Glutaminsäurerest ersetzt ist, der Glutaminsäurerest in der Position +79 durch einen Phenylalaninrest ersetzt ist und der Leucinrest in der Position +81 durch einen Asparaginsäurerest ersetzt ist.

7. Rekominanter Vektor nach einem der Ansprüche 5 und 6, **dadurch gekennzeichnet, dass** die transdominante Variante des TAT-Proteins die Sequenz aufweist, wie sie in der Identifizierung der Sequenz NO: 1 beginnend mit der Aminosäure +1 und endend bei der Aminosäure +86 gezeigt ist, in der:
(1) der Phenylalaninrest in der Position +38 durch einen Asparaginsäurerest ersetzt ist;
(2) der Threoninrest in der Position +40 durch einen Alaninrest ersetzt ist;
(3) der Lysinrest in der Position +41 durch einen Glutaminsäurerest ersetzt ist;
(4) der Isoleucinrest in der Position +45 durch einen Serinrest ersetzt ist; und/oder
(5) der Tyrosinrest in der Position +47 durch einen Argininrest ersetzt ist.

8. Rekombinanter Vektor nach einem der Ansprüche 5 bis 7, **dadurch gekennzeichnet, dass** es sich bei den Elementen, die für die konstitutive Expression erforderlich sind, um den PGK-Promotor oder die LTR des MoMuLV-Virus handelt.

9. Rekombinanter Vektor nach einem der Ansprüche 5 bis 8, **dadurch gekennzeichnet, dass** er von einem Virus abstammt, das aus Retroviren, Adenoviren und Adenovirus-assoziiertem Virus ausgewählt ist.

10. Eukaryotische Zelle, in deren Genom inseriert sind:
(a) eine erste DNA-Sequenz, die eine transdominante Variante des TAT-Proteins des HIV-Virus kodiert und unter die Kontrolle von Elementen gestellt ist, die für ihre konstitutive Expression erforderlich sind; und
(b) eine zweite DNA-Sequenz, die eine transdominante Variante des REV-Proteins des HIV-Virus kodiert und die Sequenz aufweist, wie sie in der Identifizierung der Sequenz NO: 2 beginnend mit der Aminosäure +1 und endend bei der Aminosäure +116 gezeigt ist, in der der Glutaminrest in der Position +74 durch einen Glycinrest ersetzt ist und der Leucinrest in der Position +75 durch einen Serinrest ersetzt ist und die unter die Kontrolle von Elementen gestellt ist, die für ihre konstitutive Expression erforderlich sind.

11. Eukaryotische Zelle nach Anspruch 10, **dadurch gekennzeichnet, dass** bei der transdominanten Variante des REV-Proteins darüber hinaus der Leucinrest in der Position +78 durch einen Glutaminsäurerest ersetzt ist, der Glutaminsäurerest in der Position +79 durch einen Phenylalaninrest ersetzt ist und der Leucinrest in der Position +81 durch einen Asparaginsäurerest ersetzt ist.

12. Eukaryotische Zelle nach einem der Ansprüche 10 bis 11, **dadurch gekennzeichnet, dass** die transdominante Variante des TAT-Proteins die Sequenz aufweist, wie sie in der Identifizierung der Sequenz NO: 1 beginnend mit der Aminosäure +1 und endend bei der Aminosäure +86 gezeigt ist, in der:
(1) der Phenylalaninrest in der Position +38 durch einen Asparaginsäurerest ersetzt ist;
(2) der Threoninrest in der Position +40 durch einen Alaninrest ersetzt ist;
(3) der Lysinrest in der Position +41 durch einen Glutaminsäurerest ersetzt ist;
(4) der Isoleucinrest in der Position +45 durch einen Serinrest ersetzt ist; und/oder
(5) der Tyrosinrest in der Position +47 durch einen Argininrest ersetzt ist.

13. Eukaryotische Zelle nach einem der Ansprüche 10 bis 12, **dadurch gekennzeichnet, dass** es sich bei den Elementen, die für eine konstitutive Expression erforderlich sind, um den PGK-Promotor oder die LTR des MoMuLV-Virus handelt.

14. Verwendung eines rekombinanten Vektors nach einem der Ansprüche 5 bis 9 oder einer Zelle nach einem der Ansprüche 10 bis 13 für die Herstellung eines Medikaments, das zur Behandlung oder Verhütung von auf HIV zurückzuführenden Virusinfektionen bestimmt ist.

15. Zusammensetzung nach einem der Ansprüche 1 bis 3, die darüber hinaus ein unter einem pharmazeutischen Gesichtspunkt annehmbares Vehikel umfasst.

16. Pharmazeutische Zusammensetzung, umfassend einen rekombinanten Vektor nach einem der Ansprüche 5 bis 9 oder eine Zelle nach einem der Ansprüche 10 bis 13 in Verbindung mit einem unter einem pharmazeutischen Gesichtspunkt annehmbaren Vehikel.

17. Kit, umfassend
(a) eine erste transdominante Variante eines TAT-Proteins des HIV-Virus und
(b) eine zweite transdominante Variante des REV-Proteins des HIV-Virus, **dadurch gekennzeichnet, dass** die transdominante Variante des REV-Proteins die Sequenz aufweist, wie sie in der Identifizierung der Sequenz NO: 2 beginnend mit der Aminosäure +1 und endend bei der Aminosäure +116 gezeigt ist, in der der Glutaminrest in der Position +74 durch einen Glycinrest ersetzt ist und der Leucinrest in der Position +75 durch einen Serinrest ersetzt ist;
mit Instruktionen für deren gleichzeitige oder aufeinanderfolgende Verabreichung.

## Claims

1. Composition comprising at least:
(a)a first trans-dominant variant of the TAT protein of the HIV virus; and
(b)a second trans-dominant variant of the REV protein of the HIV virus, **characterized in that** the trans-dominant variant of the REV protein has the sequence as shown in sequence identifier NO: 2 beginning at amino acid +1 and ending at amino acid +116 in which the glutamine residue at position +74 is replaced by a glycine residue and the leucine residue at position +75 is replaced by a serine residue.

2. Composition according to Claim 1, **characterized in that** the trans-dominant variant of the REV protein furthermore has the leucine residue at position +78 replaced by a glutamic acid residue, the glutamic acid residue at position +79 replaced by a phenylalanine residue and the leucine residue at position +81 replaced by an aspartic acid residue.

3. Composition according to Claim 1 or 2, **characterized in that** the trans-dominant variant of the TAT protein has the sequence as shown in sequence identifier NO: 1 beginning at amino acid +1 and ending at amino acid +86 in which:
(1) the phenylalanine residue at position +38 is replaced by an aspartic acid residue;
(2) the threonine residue at position +40 is replaced by an alanine residue;
(3) the lysine residue at position +41 is replaced by a glutamic acid residue;
(4) the isoleucine residue at position +45 is replaced by a serine residue; and/or
(5) the tyrosine residue at position +47 is replaced by an arginine residue.

4. Composition according to one of Claims 1 to 3, as a combination product for use simultaneously, separately or spread over time, intended for the treatment or prevention of infections caused by HIV.

5. Recombinant vector comprising:
(a) a first DNA sequence coding for a trans-dominant variant of the TAT protein of the HIV virus, placed under the control of the elements needed for its constitutive expression; and
(b) a second DNA sequence coding for a trans-dominant variant of the REV protein of the HIV virus having the sequence as shown in sequence identifier NO: 2 beginning at amino acid +1 and ending at amino acid +116 in which the glutamine residue at position +74 is replaced by a glycine residue and the leucine residue at position +75 is replaced by a serine residue, placed under the control of the elements needed for its constitutive expression.

6. Recombinant vector according to Claim 5, **characterized in that** the trans-dominant variant of the REV protein furthermore has the leucine residue at position +78 replaced by a glutamic acid residue, the glutamic acid residue at position +79 replaced by a phenylalanine residue and the leucine residue at position +81 replaced by an aspartic acid residue.

7. Recombinant vector according to either Claim 5 or 6, **characterized in that** the trans-dominant variant of the TAT protein has the sequence as shown in sequence identifier NO: 1 beginning at amino acid +1 and ending at amino acid +86 in which:
(1) the phenylalanine residue at position +38 is replaced by an aspartic acid residue;
(2) the threonine residue at position +40 is replaced by an alanine residue;
(3) the lysine residue at position +41 is replaced by a glutamic acid residue;
(4) the isoleucine residue at position +45 is replaced by a serine residue; and/or
(5) the tyrosine residue at position +47 is replaced by an arginine residue.

8. Recombinant vector according to one of Claims 5 to 7, **characterized in that** the elements necessary for constitutive expression are the PGK promoter or the LTR of the MoMuLV virus.

9. Recombinant vector according to one of Claims 5 to 8, **characterized in that** it is derived from a virus selected from retroviruses, adenovirus and adeno-associated virus.

10. Eukaryotic cell into the genome of which there are inserted:
(a) a first DNA sequence coding for a trans-dominant variant of the TAT protein of the HIV virus, placed under the control of the elements needed for its constitutive expression; and
(b) a second DNA sequence coding for a trans-dominant variant of the REV protein of the HIV virus, the sequence as shown in sequence identifier NO: 2 beginning at amino acid +1 and ending at amino acid +116 in which the glutamine residue at position +74 is replaced by a glycine residue and the leucine residue at. position +75 is replaced by a serine residue, placed under the control of the elements needed for its constitutive expression.

11. Eukaryotic cell according to Claim 10, **characterized in that** the trans-dominant variant of the REV protein furthermore has the leucine residue at positions +78 replaced by a glutamic acid residue, the glutamic acid residue at position +79 replaced by a phenylalanine residue and the leucine residue at position +81 replaced by an aspartic acid residue.

12. Eukaryotic cell according to either Claim 10 or 11, **characterized in that** the trans-dominant variant of the TAT protein has the sequence as shown in sequence identifier NO: 1 beginning at amino acid +1 and ending at amino acid +86 in which:
(1) the phenylalanine residue at position +38 is replaced by an aspartic acid residue;
(2) the threonine residue at position +40 is replaced by an alanine residue;
(3) the lysine residue at position +41 is replaced by a glutamic acid residue;
(4) the isoleucine residue at position +45 is replaced by a serine residue; and/or
(5) the tyrosine residue at position +47 is replaced by an arginine residue.

13. Eukaryotic cell according to one of Claims 10 to 12, **characterized in that** the elements necessary for constitutive expression are the PGK promoter or the LTR of the MoMuLV virus.

14. Use of a recombinant vector according to one of Claims 5 to 9 or a cell according to any of Claims 10 to 13 for the preparation of a medicinal product intended for the treatment or prevention of viral infections, and in particular infections caused by HIV.

15. Composition according to one of Claims 1 to 3, which comprises, in addition, a vehicle which is acceptable from a pharmaceutical standpoint.

16. Pharmaceutical composition comprising a recombinant vector according to one of Claims 5 to 9 or a cell according to one of Claims 10 to 13, in combination with a vehicle which is acceptable from a pharmaceutical standpoint.

17. Kit comprising
(a) a first trans-dominant variant of a TAT protein of the HIV virus, and
(b) a second trans-dominant variant of the REV protein of the HIV virus, **characterized in that** the trans-dominant variant of the REV protein has the sequence as shown in sequence identifier NO: 2 beginning at amino acid +1 and ending at amino acid +116 in which the glutamine residue at position +74 is replaced by a glycine residue and the leucine residue at position +75 is replaced by a serine residue;
with directions for their concomitant or sequential administration.
